(19) **Europäisches Patentamt / European Patent Office / Office européen des brevets**

(11) **EP 3 792 380 B1**

(12) # EUROPEAN PATENT SPECIFICATION

(45) Date of publication and mention
of the grant of the patent:
**20.03.2024 Bulletin 2024/12**

(21) Application number: **19800800.5**

(22) Date of filing: **09.05.2019**

(51) International Patent Classification (IPC):
**D01F 6/60** *(2006.01)* **A61L 29/06** *(2006.01)*
**A61L 29/14** *(2006.01)* **D01F 6/80** *(2006.01)*
**D07B 1/02** *(2006.01)*

(52) Cooperative Patent Classification (CPC):
(C-Sets available)
**D01F 6/605; A61L 29/06; A61L 29/14; D01F 6/805;
D07B 1/147;** D07B 2205/205; H01B 7/183  (Cont.)

(86) International application number:
**PCT/JP2019/018610**

(87) International publication number:
**WO 2019/216385 (14.11.2019 Gazette 2019/46)**

(54) **WHOLLY AROMATIC POLYAMIDE FIBER**

VOLLAROMATISCHE POLYAMIDFASER

FIBRE DE POLYAMIDE ENTIÈREMENT AROMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.05.2018 JP 2018091379**

(43) Date of publication of application:
**17.03.2021 Bulletin 2021/11**

(73) Proprietor: **Teijin Limited**
**Osaka 530-0005 (JP)**

(72) Inventors:
• **KOMIYA, Naoya**
**Osaka-shi, Osaka 530-0005 (JP)**
• **KAWAMURA, Kenji**
**Osaka-shi, Osaka 530-0005 (JP)**

(74) Representative: **Carpmaels & Ransford LLP**
**One Southampton Row**
**London WC1B 5HA (GB)**

(56) References cited:
| | |
|---|---|
| EP-A1- 0 051 265 | JP-A- H0 576 480 |
| JP-A- S6 189 317 | JP-A- H06 319 685 |
| JP-A- H11 189 916 | JP-A- 2006 299 476 |
| JP-A- 2008 156 802 | JP-A- 2010 150 699 |
| JP-A- 2011 026 726 | JP-A- 2013 170 334 |
| JP-A- 2014 015 705 | JP-A- 2014 070 306 |
| JP-A- 2014 105 404 | JP-A- 2015 098 664 |
| JP-A- 2015 193 730 | JP-A- 2016 176 161 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 29/06, C08L 77/10;**
D07B 2205/205, D07B 2801/10

**Description**

FIELD

The present disclosure relates to a wholly aromatic polyamide fiber. More particularly, the present disclosure relates to a wholly aromatic polyamide fiber in which both fibers and single yarns are thin and flexible, and which is excellent in handling property. The present disclosure also relates to a composite cable and a catheter using such a wholly aromatic polyamide fiber.

BACKGROUND

Conventionally, a composite cable for signal transmission is used for transferring data between electronic devices. In recent years, with the sophistication of the electronic devices, the amount of data to be transmitted has increased remarkably, and high-speed transmission has been demanded. In order to satisfy such requirements, signal transmission lines constituting the composite cable is increased so that a large amount of data is transmitted in a short time.

For such a composite cable, from the viewpoint of handling property, thin and flexible composite cable is required and thus the reduction of the diameter of the composite cable is desired. When the composite cable is reduced in diameter, the tensile strength of the composite cable itself is naturally reduced greatly. Therefore, it is important to arrange reinforcing fibers to prevent disconnection of the transmission line of the composite cable. However, the space in which the reinforcing fibers are arranged is a gap between the transmission lines. Therefore, with the reduction in the diameter of the composite cable, the space for the arrangement of the reinforcing fibers has become extremely small.

Therefore, a thin reinforcing fiber that fits in such an extremely small space is strongly required.

In addition, with the development of advanced catheter surgical methods in recent years, advancement of various types of equipment are required. In particular, there is a need for a catheter that is thin, light, and stronger. The catheter is also required to have morphological retention, such as flexibility, collapse resistance and kink resistance, as well as burst resistance, airtightness, and chemical resistance. However, the polymer alone could not sufficiently satisfy such requirements. In order to satisfy these characteristics, it is conceivable to arrange reinforcing fibers in the resin layer of the tube outer layer, for example. However, the conventional fiber has a problem that the thickness is relatively large due to relatively thick single yarn, that the flexibility is low, and that the kinks occur. The primary problem with such fibers was that the thin catheter could not be formed.

Thus, there is a need for thin fibers to reinforce products such as composite cable and catheter requiring thinness.

In Patent Document 1, a relatively thin wholly aromatic polyamide fiber is proposed. This document describes aramid fibers consisting of aramid multi-filaments having a single yarn fineness of 3.5 to 10 dtex and a total fineness of 10 to 100 dtex, and comprising 0.3 to 5.0 % of oil agent adhered thereto.

[RELATED ART]

[PATENT LITERATURE]

[Patent Document 1] JP-A-2015-98665

EP 0 051 265 A1 describes aromatic polyamide fibers from aromatic polyamides whose chain-extending bonds are either coaxial or parallel and oppositely directed, wherein the fibers are obtained by dry spinneret wet spinning downwardly into a shallow coagulating bath having an orifice in its bottom for removal of coagulating liquid and fibers wherein no more than a minor portion of coagulating liquid lower than the entrance of the bath orifice in the proximity of the bath orifice.

JP 2008 156802 A describes a rope constituted of wholly aromatic polyamide fibers, particularly copoly-p-phenylene 3,4' -oxydiphenylene-terephthalamide fibers in which 1.5-14 mg/m² of inorganic fine powder having an average particle diameter of about ≤20 μm is added to the fiber surface.

JP H11 189916 A describes an aramide fiber obtained by spinning a sulfuric acid solution of an aromatic polyamide such as poly(p-phenylene terephthalamide) into a coagulation bath through a nozzle and an air gap, washing the spun fiber with water, neutralizing the washed fiber, and subsequently drying the treated fiber.

SUMMARY

[PROBLEM TO BE SOLVED BY THE INVENTION]

Since a conventional wholly aromatic polyamide fiber has a relatively thick single yarn fineness, there has been a problem that it is difficult to arrange many fibers composed of multiple single yarns in a limited space, and that a single yarn has poor flexibility.

The invention according to the present disclosure has been made in view of such problems, and an object thereof is to provide a wholly aromatic polyamide fiber having a reduced total fineness and a reduced single yarn fineness, but also excellent both in quality and reinforcing property.

[SOLUTION TO THE PROBLEM]

The inventor of the present invention has intensively studied to solve the above-mentioned problem, and has found that the above-mentioned problem can be solved by, in a fiber having a particular physical property, suppressing the number of fibrils on a single yarn surface to a predetermined value or less, and has completed the present invention.

The wholly aromatic polyamide fiber of the present disclosure is defined in the claims.

The invention according to the present disclosure further includes a composite cable characterized in that it comprises the above-mentioned wholly aromatic polyamide fiber. Further, the invention according to the present disclosure includes a catheter characterized in that it comprises the above-mentioned wholly aromatic polyamide fiber.

According to the present disclosure, there is provided a wholly aromatic polyamide fiber which has a reduced total fineness and a reduced single yarn fineness, while also having excellent quality and reinforcing property.

Since the wholly aromatic polyamide fiber according to the present disclosure has a reduced diameter while maintaining tensile strength, flexibility and kink resistance, it can be applied to an extremely thin composite cable, a catheter, or the like, as a reinforcing fiber for example.

BRIEF DESCRIPTION OF THE DRAWINGS

[Figure 1] FIG. 1 is a schematic cross-sectional view of a composite cable according to the present disclosure.
[Figure 2] FIG.2 is a schematic cross-sectional view of a catheter according to the present disclosure.
[Figure 3] FIG.3 is a schematic view of a fiber wound as a cheese roll on a paper tube.

DESCRIPTION OF EMBODIMENTS

Hereinafter, embodiments of the present disclosure will be described in detail.

< <Wholly aromatic polyamide fiber>>

Wholly aromatic polyamide fiber of the present disclosure is characterized in that: a single yarn fineness is 0.4 dtex to 3.5 dtex, a total fineness is 5 dtex to 30 dtex, a breaking strength is 15 cN/dtex or more, an initial tensile modulus is 500 cN/dtex to 750 cN/dtex, and the number of fibrils is less than 100/m.

Generally, a wholly aromatic polyamide fiber comprises a plurality of single yarns in bundles.

In the conventional wholly aromatic polyamide fiber, it comprised a relatively thick single yarn for reducing the fiber diameter and preventing yarn breakage. However, when the single yarn fineness is relatively thick, there is a problem that the reduction of the total fineness is limited and the desired flexibility cannot be obtained. In other words, when the single yarn fineness is relatively thick, it is difficult to arrange many fibers composed of multiple single yarns in a limited space, and the flexibility of the single yarn may be poor.

Further, according to the prior art, a reinforcing fiber having a reduced total fineness and a reduced single yarn fineness may cause a decrease in physical properties of the fiber during manufacturing process and a breakage of a single yarn during processing process, and is not suitable as reinforcing fiber.

On the other hand, the present inventors have found that, by reducing fibrils on the surface of single yarns in a fiber having a particular physical property, single yarn breakage can be suppressed while maintaining a reduced single yarn fineness.

Although there is no intention to limit the invention by theory, since a thinner single yarn has a larger specific surface area, the physical properties of the single yarn surface would have greater influence on the breakability of the single yarn.

In the wholly aromatic polyamide fiber according to the present disclosure, it can be considered that, by reducing the number of fibrils on the surface of a single yarn to a predetermined number or less, physical properties of the surface of the single yarn are improved, and as a result, the single yarn breakage is hardly caused even when the single yarn fineness is relatively low.

Furthermore, the wholly aromatic polyamide fiber according to the present disclosure have relatively high flexibility because it has relatively thin single yarns.

Therefore, according to the present disclosure, it is possible to provide a wholly aromatic polyamide fiber having a reduced total fineness and a reduced single yarn fineness, while also having excellent quality and reinforcing property.

<Breaking strength>

The breaking strength of the wholly aromatic polyamide fiber of the present disclosure needs to be 15 cN/dtex or more, preferably 18 cN/dtex or more, more preferably 20 cN/dtex or more, still more preferably 22 cN/dtex or more, particularly preferably 23 cN/dtex or more, more particularly preferably 24 cN/dtex or more, and most preferably 25 cN/dtex or more.
Wholly aromatic polyamide fiber having the breaking strength of 15 cN/dtex or more exhibits excellent tensile strength, and is suitable for example as the reinforcing fiber of the composite cable.

<Initial tensile modulus>

The initial tensile modulus of the wholly aromatic polyamide fiber of the present disclosure needs to be from 500 cN/dtex to 750 cN/dtex, preferably from 520 cN/dtex to 730 cN/dtex, and more preferably from 550 cN/dtex to 700 cN/dtex.
When the initial tensile modulus is 500 cN/dtex or more, tensile strength can be maintained. Specifically, for example, when the fiber of the present disclosure is used as a reinforcing fiber of a composite cable, it is possible to suppress the elongation of fibers, even when an instantaneous stress is applied to the composite cable.
On the other hand, when the initial tensile modulus is 750 cN/dtex or less, flexibility and kink resistance of the yarn are secured, and fibrillation in the processing step can be suppressed.

<Number of fibrils>

It is necessary that the number of fibrils in a single yarn of the wholly aromatic polyamide fiber of the present disclosure is less than 100/m, preferably less than 70/m, more preferably less than 50/m, still more preferably less than 30/m, particularly preferably less than 25/m, and most preferably less than 20/m.
When the number of fibrils is less than 100/m, it is possible to suppress the entanglement of single yarns on a guide or the like during the processing step, and as a result, it is possible to suppress the occurrence of yarn breakage.

<Elongation at break>

The elongation at break of the wholly aromatic polyamide fiber of the present disclosure is preferably from 3.0 % to 6.0 %, more preferably from 3.5 % to 5.5 %, and still more preferably from 4.0 % to 5.0 %.
When the elongation at break is 3.0 % or more, the single yarn becomes difficult to break against peeling or stress at the adhesive interface. When the elongation at break is 6.0 % or less, elongation of the fiber at the time of stress can be suppressed, and tensile strength can be maintained.

<Single yarn fineness>

The single yarn fineness of the wholly aromatic polyamide fiber of the present disclosure needs to be from 0. 4 dtex to 3. 5 dtex, preferably from 0. 4 dtex to less than 3. 5 dtex, more preferably from 0. 6 dtex to 3. 0 dtex, still more preferably from 0. 7 dtex to 2. 5 dtex, and particularly preferably from 0. 8 dtex to 2.0 dtex.
The single yarn fineness of 0. 4 dtex to 3. 5 dtex allows a relatively large number of single-yarn fibers to be filled into a confined space in a composite cable having reduced diameter.
When the single yarn fineness is 0.4 dtex or more, it is ensured that the single yarn has sufficient strength to suppress the single yarn breakage. When the single yarn fineness is 3 5 dtex or less, it becomes unnecessary to enhance the orientation and crystallinity of the skin layer for increasing the breaking strength and the initial tensile modulus, and as a result, fibrillation can be suppressed.

<Total fineness>

The total fineness of the wholly aromatic polyamide fiber of the present disclosure needs to be from 5dtex to 30 dtex, preferably from 10 dtex to 25 dtex, and more preferably from 15 dtex to 20 dtex.
When the total fineness is 30 dtex or less, the cross-sectional area of the fiber as a whole can be reduced, and the fiber can be arranged in an extremely small space in a composite cable having reduced diameter. Further, when the total fineness is 30 dtex or less, the thickness of the catheter tube can be reduced as compared with a conventional one.
When the total fineness is 5 dtex or more, it is possible to ensure that the fiber has sufficient strength to suppress the yarn breakage during the processing step.

<Wholly aromatic polyamide>

The wholly aromatic polyamide constituting the wholly aromatic polyamide fiber of the present disclosure is a polyamide in which one type of or two or more types of divalent aromatic groups are directly linked through amide bonds. Aromatic groups include those in which two aromatic rings are bonded via oxygen, sulfur, or alkylene group, or those in which two or more aromatic rings are directly bonded. Further, these divalent aromatic groups may comprise lower alkyl group such as methyl group or ethyl group, methoxy group, or halogen group such as chloro group, and the like.

Such wholly aromatic polyamides may include, for example, polyparaphenylene terephthalamide; polymetaphenylene isophthalamide; copolyparaphenylene 3,4'-oxydiphenylene terephthalamide in which the terephthalic acid component, the 3,4'-diaminodiphenyl ether component and the paraphenylenediamine component are copolymerized; and copolyparaphenylene phenylbenzimidazole terephthalamide in which the terephthalic acid component, the aromatic diamine component with the phenylbenzimidazole backbone, and the paraphenylenediamine component are copolymerized. In the context of the production method according to the present invention, the above-mentioned wholly aromatic polyamides may be used alone, or two or more of them may be used in combination.

From the viewpoint of exhibiting high mechanical properties in the dry and wet method, the wholly aromatic polyamide fiber of the present disclosure is preferably mainly composed of the wholly aromatic polyamide. The term "mainly composed of" means that the component in question is in the range of more than 50 % by mass and not more than 100 % by mass, based on the entire wholly aromatic polyamide fiber obtained. In the present invention, it is particularly preferred that the para-type aromatic polyamide is 100 % by mass, based on the entire wholly aromatic polyamide fiber.

Further, in the present disclosure, since the mechanical strength is particularly excellent, it is preferable to use polyparaphenylene terephthalamide or copolyparaphenylene 3, 4'-oxydiphenylene terephthalamide as the wholly aromatic polyamide. Further, it is most preferable to use copolyparaphenylene 3,4 '-oxydiphenylene terephthalamide as the wholly aromatic polyamide, since it is excellent in molding workability due to its solubility in amide-based solvent or the like, and since the tensile properties such as the strength and the initial tensile modulus can be remarkably improved by subjecting it to hot-drawing.

In one embodiment of the present disclosure, the wholly aromatic polyamide fiber of the present disclosure is a polyparaphenylene terephthalamide fiber.

In yet another embodiment of the present disclosure, the wholly aromatic polyamide fiber of the present disclosure is a copolyparaphenylene 3, 4'-oxydiphenylene terephthalamide fiber.

<Method for producing wholly aromatic polyamide fiber>

The wholly aromatic polyamide fiber of the present disclosure can be produced by a so-called dry and wet spinning method. The "dry and wet spinning method" is a method in which a spinning solution (dope) containing a wholly aromatic polyamide and a solvent is spun from a spinneret into the inert gas called air gap and is then brought into contact with a coagulation liquid to form an unstretched yarn (coagulated yarn), and then the coagulated yarn is washed with water and then is subjected to hot-drawing to obtain a fiber.

(Spinning solution (dope))

The spinning solution (dope) used in producing the wholly aromatic polyamide fiber of the present disclosure includes a wholly aromatic polyamide and a solvent. A method of preparing the spinning solution (dope) is not particularly limited, and known methods can be employed.

Examples of the solvent used for preparing the spinning solution (dope) include N-methylpyrrolidone (NMP), dimethylacetamide (DMAc), dimethylformamide (DMF), dimethyl sulfoxide (DMSO), and N-methylcaprolactam (NMC). One type of solvent may be used alone, or a mixed solvent obtained by mixing two or more types of solvents may be used. Further, a solvent used for polymerization of the wholly aromatic polyamide may be used as it is.

Further, other optional components such as an additive may be blended, for the purpose of imparting functionality and the like to the fiber. Other optional components may be introduced in the preparation of the spinning solution (dope). The method of introduction is not particularly limited. For example, other optional components can be introduced into the dope using a loader, a mixer, or the like.

Note that the polymer concentration in the spinning solution (dope), i.e., the concentration of the wholly aromatic polyamide, is preferably in the range of 1.0 % by mass or more and 10% by mass or less.

When the polymer concentration is 1.0 % by mass or more in the spinning solution (dope), entanglement of the polymer sufficient to obtain a viscosity necessary for the spinning can be ensured, and as a result, ejection stability during the spinning is improved. On the other hand, when the polymer concentration is 10 % by mass or less, it is possible to suppress the decrease in the ejection stability during the spinning cause by the abrupt increase in the viscosity of the dope.

(Spinning step)

In the spinning step, the spinning solution (dope) is ejected from a spinneret. The hole diameter, nozzle length, material, and the like of the spinneret used are not particularly limited, and can be appropriately adjusted in consideration of the spinnability and the like. The hole diameter of the nozzle is not particularly limited, but from the viewpoint of ejection stability, it is preferable that the shear stress when the dope is ejected from the spinneret is less than 17,000 Pa.

The length of the air gap to the surface of the coagulation liquid is not particularly limited, but is preferably in the range of 5 mm to 30 mm from the viewpoints of temperature controllability, spinnability, and the like.

The temperature of the polymer dope at the time of passing through the spinneret and the temperature of the spinneret are not particularly limited, but are preferably set to 60°C to 120°C from the viewpoint of the spinnability and the ejection pressure of the polymer dope.

(Coagulation step)

In the coagulation step, the spinning solution (dope) spun in the spinning step is coagulated by bringing it into contact with a coagulation liquid consisting of a poor solvent, in order to obtain an unstretched yarn (coagulated yarn).

The coagulation liquid is an aqueous solution of an amide-based solvent, and an example thereof includes an aqueous NMP (N-methylpyrrolidone) solution. The temperature of the coagulation liquid and the concentration of the amide-based solvent are not particularly limited. The temperature of the coagulation liquid and the concentration of the amide-based solvent can be appropriately adjusted within the range in which there is no problem in the coagulation state of the formed coagulation yarn, the passage of the subsequent step, and the like.

Although there is no particular limitation on the coagulation device, a device capable of suppressing a liquid flow and a swing of a tow is preferred.

As described above, in the wholly aromatic polyamide fiber of the present disclosure, a single yarn fineness is 0. 4 dtex to 3. 5 dtex, a total fineness is 5 dtex to 30 dtex, a breaking strength is 15 cN/dtex or more, and an initial tensile modulus is 500 to 750 cN/dtex.

Such fibers can be obtained by controlling the running speed of yarns in a coagulation bath, e.g., by a dry-wet spinning method.

By controlling the running speed of the yarns in the coagulation bath, disturbance of the liquid flow in the bath due to the wake caused by the running yarn is prevented. Thus, it becomes possible to stably run the coagulated yarn, and as a result, it is possible to obtain a wholly aromatic polyamide fiber having the above physical properties.

The maximum tension of the spun yarn in the coagulation bath is preferably 0.1 g/dtex or more and less than 1.0 g/dtex, more preferably 0.2 g/dtex or more and less than 0.9 g/dtex, and most preferably 0.3 g/dtex or more and less than 0.8 g/dtex.

When the maximum tension of the spun yarn in the coagulation bath is less than 1.0 g/dtex, it is possible to suppress the excess orientation of the polymer molecules on the surface of the single yarn, and as a result, it is possible to suppress fibrillation of the fibers. When the maximum tension of the spun yarn in the coagulation bath is 0.1 g/dtex or more, the swinging of the single yarn in the coagulation bath is suppressed, and as a result, the frequency of occurrence of the adhesion and the variation in the diameter of the single yarn are reduced.

(Water washing step)

In the water washing step, the unstretched yarn (coagulated yarn) formed by coagulation in a coagulation bath is washed with water to remove the solvent. The temperature of the liquid used as the washing water as well as the concentration of the amide-based solvent (NMP) are not particularly limited.

(Drying step)

Next, in the drying step, the unstretched yarn (coagulated yarn) is subjected to drying. The drying condition is not particularly limited as long as the fiber can be sufficiently dried. However, considering the work efficiency and the deterioration of the fiber due to heat, it is preferable to set the temperature in the range of 150 °C to 250 °C.

Preferable drying devices include a drying device of a roller type which rotates at a speed equivalent to the speed of the yarn, or a drying device of a non-contact type, for example, a drying device of a non-contact type in which the fibers are passed through an oven. When the contact type drying apparatus such as a hot plate is used, a fibril may be generated on the surface of the single yarn by friction.

(Hot-drawing step)

Then, in the hot-drawing step, the fiber after drying is thermally stretched. Through this step, the polymer molecules in the fiber are highly oriented by thermal stretching of the fibers, and the strength is imparted to the fiber.
The hot-drawing temperature in this step is preferably in the range of 300 °C to 600 °C, more preferably in the range of 320 °C to 580 °C, and most preferably in the range of 350 °C to 550 °C.
By having a hot-drawing temperature of 600 °C or less, thermal decomposition of the polymer is suppressed, so that deterioration of the fibers is suppressed, and as a result, a yarn having high strength can be obtained.
The stretching ratio in the hot-drawing step is preferably 5 to 15 times, but is not particularly limited to this range as long as the desired strength can be achieved. In addition, this hot-drawing step may be performed in multiple stages as necessary.
The hot-drawing device is preferably either a roller-type hot-drawing apparatus which rotates at a speed equivalent to that of the yarn, or a non-contact type hot-drawing apparatus in which the fibers do not contact a furnace as they pass through the furnace. When a contact type hot-drawing device such as a hot plate is used, a fibril may be generated on the surface of the single yarn by friction.

(Oil application step)

In order to impart charge suppression and lubricity to the fiber, an oil agent is applied to the fiber, and finally, it is wound up with a winder.
Regarding the type of the oil agent and the amount thereof, the ester component contained in the oil agent component is preferably 0.3 wt% or more, more preferably 0.3 wt% to 5.0 wt%, still more preferably 0.4 wt% to 4.0 wt%, and particularly preferably 0.5 wt% to 3.0 wt%.
When the ester component contained in the oil agent component is 0.3 wt% or more, the lubricity becomes relatively high. When the ester component contained in the oil agent component is 5.0 wt% or less, generation of scum in the processing step can be suppressed, and as a result, generation of fibrils can be suppressed.

(Winding step)

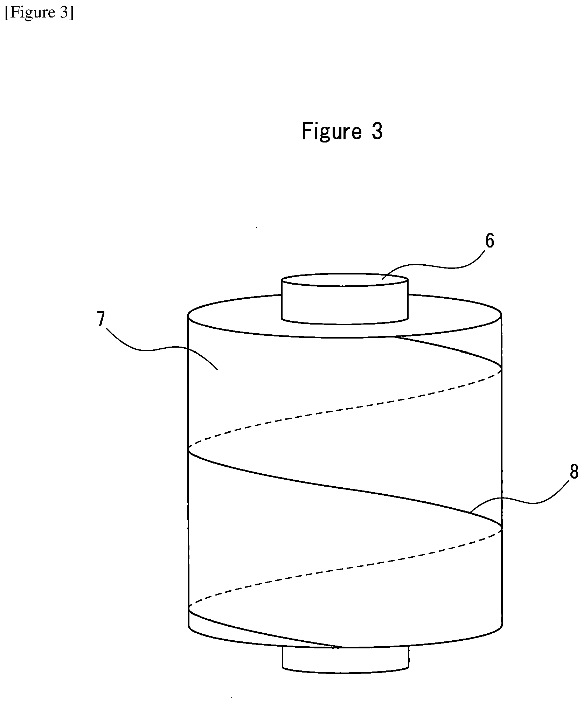

In the winding process, the fiber can be wound onto a paper tube or the like with a known winder, by appropriately adjusting the winding conditions. The wind ratio is preferably 2 to 10, more preferably 3 to 8, and still more preferably 4 to 6.
When the wind ratio is 2 or more, it is possible to suppress the occurrence of friction between the fibers during the winding, and as a result, it is possible to suppress the occurrence of fibrils. When the wind ratio is 10 or less, it is possible to prevent fibers from gathering at both ends of a bobbin (paper tube), so that it is possible to suppress the occurrence of friction between the fibers and a contact roller, and as a result, it is possible to suppress the occurrence of fibrils.
In the context of the present disclosure, "wind ratio" refers to the number of times to wind the fiber from one end face to the other end face of the cheese roll, which is shown in FIG. 3.

<Variation in single yarn diameter>

In one embodiment according to the present disclosure, a variation in the single yarn diameter per 1m of the wholly aromatic polyamide fibers according to the present disclosure is less than 15%.
Digital microscope (model VHX-2000, manufactured by KEYENCE CORPORATION) is used to measure the single yarn diameter at 10 points for every 10cm of a single yarn at a magnification of 10000 times, and the coefficient of variation CV of the single yarn diameter is calculated according to the formula shown below. The CV is calculated for 10 single yarns, and the smallest coefficient of variation CV is used as the variation of the single yarn diameter per 1m.

$$CV = \text{standard deviation/mean value} \times 100(\%)$$

The variation of the single yarn diameter per 1m is preferably less than 15 %, more preferably less than 12 %, still more preferably less than 10 %, particularly preferably less than 8 %, and most preferably less than 5 %.
The variation in the diameter of the single yarn per 1m of 15 % or more indicates that the difference between the thick portion and the thin portion of the single yarn is large. It is preferable to suppress the variation in the diameter of the single yarn per 1m to less than 15 % because it reduces the relatively thick portion of single yarn and the relatively thin portion of single yarn.

If the variation in single yarn diameter per 1m is less than 15 %, the reduction in the relatively thick portions of single yarn allows the end products such as composite cables and catheters to be relatively thin, and allows the fibers to be inserted into relatively small spaces. On the other hand, by reducing the relatively thin portion of single yarn, the weak point of the single yarn are reduced and, as a result, the cause of yarn breakage is reduced.

Note that this variation in the single yarn diameter can be more stably achieved by reducing the number of fibrils.

<Placeability of the fiber>

In one embodiment of the present disclosure, the average value of the thickness of the twisted fiber, which is calculated as follows, is less than 40 $\mu$m:

The average value of the thickness of the twisted fiber is calculated by twisting the fiber the number of times calculated based on the following equation (2) using the weight calculated based on the following equation (1), by measuring the thickness of the twisted fiber in five points every 10cm in the length direction of the fiber, and by calculating the average of the measured values.

$$\text{Weight (g)=fineness (dtex)/40} \qquad (1)$$

$$\text{Number of twists (times/m)} = 1055/\sqrt{\text{fineness (tex)}}. \qquad (2)$$

The average thickness of the twisted fiber may be 40 $\mu$m or less, 30 $\mu$m or less, 25 $\mu$m or less or 20 $\mu$m or less, and/or 5 $\mu$m or more, 10 $\mu$m or more or 15 $\mu$m or more.

When the average value of the thickness of the twisted fiber is less than 40 $\mu$m, a final product such as composite cables and catheters can be made relatively thin and it is possible to insert the fiber into a relatively narrow space.

Note that, in the context of the present disclosure, the average value of the thickness of the twisted fiber is used as an indicator of the placeability of the fiber (i.e., as an index of ease of placement). In other words, when the average value of the thickness of the twisted fiber is less than 40 $\mu$m, the placeability of the fiber is judged to be good. On the other hand, when the average value of the thickness of the twisted fiber is 40$\mu$m or more, the placeability of the fiber is judged to be poor.

<Average degree of adhesion>

In one embodiment according to the present disclosure, the average degree of adhesion of the wholly aromatic polyamide fiber according to the present disclosure is 10 or less.

The average degree of adhesion is measured by a similar hooking method as in JIS L-1013: 2010 8.15 (Determination of the degree of confounding).

Specifically, the average degree of adhesion is measured as described below.

One end of the fiber sample is attached to the upper grip of a hanging device with suitable functionality, the weight is suspended to a position below 1m from the grip, and the sample is hanging vertically. The load of the weight shall be obtained by multiplying the number of display tex of the sample by 17.64 (mN), and shall be limited to 980 mN. Then, a load obtained by dividing the number of texes of the sample by the number of filaments and then by multiplying the resultant value by 88.2 (lower limit of the load: 19.6 mN, upper limit: 98 mN) is attached to one end of the hook, and the other end of the hook is inserted into a portion obtained by dividing the fiber sample into two, and then the hook is lowered. If the stopped part is confounded, it is excluded from the measurement data. The lowering distance of the hook is determined from the point where the hook stops due to adhesion between single yarns, and the degree of adhesion S is calculated by the following equation. The average value of five trials is defined as the average degree of adhesion.

$$S=1000/L$$

S: Degree of adhesion
L: Lowering distance of the hook (mm)

In the present disclosure, the average degree of adhesion is preferably 10 or less, more preferably 5 or less, still more preferably 4 or less, and particularly preferably 3 or less.

When the average degree of adhesion is 10 or less, it is possible to prevent the fiber from taking the form of single monofilament. This is preferable because the fiber can be inserted into a narrow space and the tearing of the fiber for the thickness reduction can be performed well.

<<Composite cable>>

The present disclosure also relates to a composite cable, which is characterized in that it comprises the wholly aromatic polyamide fiber according to the present disclosure.

In the context of the present disclosure, the "composite cable" refers to a cable in which a plurality of multi-purpose cables are grouped into a single cable for the purpose of transmitting and receiving data. The "cable" refers to an optical fiber capable of high-speed transmission, or a metal wire for transmitting a power source, a low-speed control signal, or the like. These components are combined with the reinforcing material to form a composite cable.

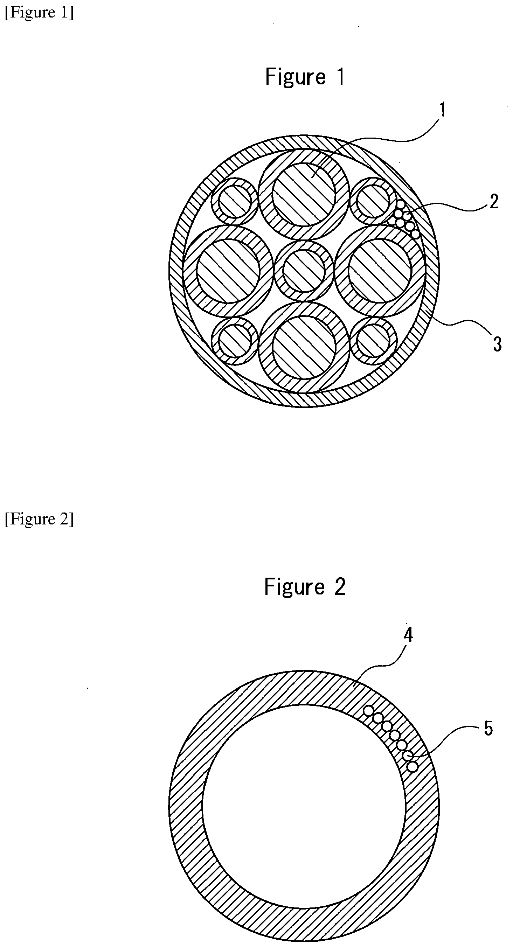

FIG. 1 is a schematic cross-sectional view of a composite cable according to the present disclosure. As shown in FIG. 1, the wholly aromatic polyamide fibers of the present disclosure, which have been subjected to fireproof treatment or twisting, are arranged along one or more cables (metal wires in FIG. 1) so that they reinforce the cables by filling gaps between the cables. The reinforced cable is coated with a coating resin.

For the composite cable, space saving, improvement in machining efficiency, and downsizing are desired. The reinforcing material used in the composite cable needs to be thin and flexible, and needs to be difficult to disconnect. Glass fibers, aramid fibers, carbon fibers, and the like are used as the reinforcing fibers for the reinforcing material, but in terms of flexibility, the wholly aromatic polyamide fiber of the present disclosure is effective.

In other words, the wholly aromatic polyamide fiber according to the present disclosure has a reduced total fineness and a reduced single yarn fineness, while also having excellent quality and reinforcing property. Therefore, the wholly aromatic polyamide fiber according to the present disclosure is suitable as a reinforcing material used in the composite cable for signal transmission.

The use of the wholly aromatic polyamide fiber of the present disclosure enables the formation of thinner composite cable and thus makes it possible to downsize the composite cable, and also provides a composite cable that is less susceptible to disconnection.

< <Catheter> >

The present disclosure also relates to a catheter characterized in that it comprises the wholly aromatic polyamide fiber according to the present disclosure.

In the context of the present disclosure, a "catheter" is a thin medical tube made of plastic, rubber, metal or the like, which is inserted into a body cavity, an organ in the body or the like, and which is used for draining or collecting content, infusing a medical fluid or a contrast agent, or measuring pressure.

Such medical catheters are generally made of nylon, polyurethane, a mixture of polyether and polyamide, or other polymeric materials.

FIG.2 is a cross-sectional schematic view of the catheter according to the present disclosure. The catheter according to the present disclosure is formed by arranging the wholly aromatic polyamide fibers of the present disclosure, which have been for example cylindrically knitted, imparted with non-combustibility, or twisted, in a coating resin layer of the catheter as shown in FIG. 2.

In most cases, for obtaining a desired function of a catheter, the catheter needs to have an ability to pass through a relatively tortuous passageway, such as a series of blood vessels in the patient's body. Thus, a catheter that has a high degree of potential for torque in the longitudinal direction while maintaining a high degree of flexibility is desired. One way to provide a catheter with the desired flexibility and torque is to arrange reinforced fibers in the wall of the catheter.

As such a reinforcing material, glass fiber, aramid fiber, carbon fiber, or the like is conceivable, but it is necessary to have flexibility from the viewpoint of not causing damage to a tube such as a blood vessel of a patient through which a catheter passes. In terms of flexibility, the wholly aromatic polyamide fiber of the present disclosure is effective.

In other words, the wholly aromatic polyamide fiber according to the present disclosure has a reduced total fineness and a reduced single yarn fineness, while also having excellent quality and reinforcing property. Further, the wholly aromatic polyamide fiber according to the present disclosure has a relatively high flexibility due to relatively thin single yarns. Therefore, the wholly aromatic polyamide fiber according to the present disclosure is suitable as a reinforcing fiber of a catheter having a thin reinforcing layer.

The use of the wholly aromatic polyamide fiber of the present disclosure enables the formation of thinner catheter and thus makes it possible to downsize the catheter, and also makes it possible to obtain a catheter which is hard to disconnect but has good flexibility.

EXAMPLES

Hereinafter, the present invention will be described more specifically by way of Examples and Comparative Examples, but the present invention is not limited in any way by these descriptions.

<<Preparation and evaluation of wholly aromatic polyamide fibers>>

Wholly aromatic polyamide fibers according to Examples 1 to 10 and Comparative Examples 1 to 6 were prepared. The wholly aromatic polyamide fibers according to Examples 1 to 7 and Comparative Examples 1 to 4 were copolyparaphenylene 3, 4'-oxydiphenylene terephthalamide fibers. The wholly aromatic polyamide fibers according to Examples 8 to 10 and Comparative Examples 5 to 6 were polyparaphenylene terephthalamide fibers. For each of the obtained fibers, the total fineness, the number of single yarns, the single yarn fineness, the breaking strength, the initial tensile modulus, the elongation at break, the number of fibrils, the variation in the single yarn diameter, the fiber placeability, and the average degree of adhesion were measured and evaluated.

<Methods for measurement and evaluation>

The characteristics of Examples and Comparative Examples were measured and evaluated by the following methods.

(Total fineness)

The obtained fibers were wound up by 100 m using a known measuring machine, and the mass thereof was measured. The total fineness was obtained by multiplying the measured weight by 100; that is, the total fineness was the fineness (dtex) per 10,000 m.

(Single yarn fineness)

The single yarn fineness was calculated by dividing the total fineness of the obtained fibers by the number of single yarns.

(Breaking strength, elongation at break, initial tensile modulus)

The breaking strength and the elongation at break were measured under the following conditions, based on ASTM D885 procedure, by using a tensile testing device (trade name: INSTRON, model: 5565, manufactured by INSTRON) and by using a chuck for yarn test. Further, the initial tensile modulus was also calculated based on ASTM D885.
Measurement conditions:

Temperature: room temperature
Specimen: 75 cm
Test speed: 250 mm/min
Distance between chucks: 500 mm

(Number of fibrils)

One meter of single yarns were randomly extracted from fibers, and for 10 single yarns, the surface thereof was observed at a magnification of 10000 times by a digital microscope (model VHX-2000, manufactured by KEYENCE CORPORATION), and the number of fibrils with a length of 2 $\mu$m or more was counted. The value obtained by dividing this count value by 10 was defined as the number of fibrils (number/m).

(Variation in single yarn diameter)

For one single yarn, diameter of a single yarn was measured at 10 points every 10cm by a digital microscope (model VHX-2000, manufactured by KEYENCE CORPORATION) at a magnification of 10000 times, and the coefficient of variation CV thereof was calculated. CV was calculated for 10 single yarns, and the smallest coefficient of variation CV was defined as the variation of the single yarn diameter per 1m.

$$CV = \text{standard deviation/mean value} \times 100(\%)$$

(Fiber placeability)

The fibers were tensioned using a weight calculated based on Equation (1), and were twisted a number of times calculated based on Equation (2). Afterwards, the thickness of the twisted fiber was measured by a micrometer (trade name: Coolant Proof (trademark) micrometer, manufactured by Mitutoyo Co.) at 5 points per 1m along the length direction, and the average thickness of the twisted fiber was calculated.

$$\text{Weight (g)=fineness (dtex)}/40 \qquad (1)$$

$$\text{Number of twists (times/m)} = 1055/\sqrt{\text{fineness (tex)}} \qquad (2)$$

If the average value of the thickness of the twisted fiber was less than 40 $\mu$m, it was determined that the fiber placeability was good. When the average value of the thickness of the twisted fiber was 40 $\mu$m or more, it was determined that the fiber placeability was poor.

(Average degree of adhesion)

A degree of adhesion was measured by a hooking method similar to the method for measuring the degree of confounding described in JIS L-1013: 2010 8.15. One end of a fiber sample was attached to an upper grip of a suitable hanging device, the weight was suspended at a position 1m below the grip, and the sample was hanging vertically. The weight load was a load (mN) obtained by multiplying the display tex number of the sample by 17.64, and the limit was set to be 980 mN. At one end of the hook, a load of 88.2 times the number of texes of the sample divided by the number of filaments (lower limit 19.6 mN, upper limit 98 mN) was applied, and the other end of the hook was inserted at the point where the fiber was divided into two, and the hook was lowered. Based on the lowering distance of the hook to the point where the hook was stopped by the adhesion between the single yarns, the degree of adhesion S was calculated by the equation shown below. The average value of five trials was defined as the average degree of adhesion. Incidentally, when the point where the hook stopped had been entangled, it was excluded from the measurement data.

$$S=1000/L$$

S: Degree of adhesion
L: Lowering distance of the hook (mm)

<Example 1>

(Preparation of the dope)

A polymer solution (dope) of copolyparaphenylene 3, 4'-oxydiphenylene terephthalamide was prepared according to a known method. Specifically, 100 parts by mass of terephthalic acid dichloride was added to an NMP in which 50 parts by mass of paraphenylenediamine and 50 parts by mass of 3,4 '-diaminodiphenyl ether were dissolved, and a polycondensation reaction was performed to obtain a polymer solution (dope) of copolyparaphenylene 3, 4'-oxydiphenylene terephthalamide. The polymer concentration was 6% by mass. The intrinsic viscosity (IV) of the polymer was 3.38.

(Production of wholly aromatic polyamide fibers)

The polymer solution (dope) obtained above was ejected using a spinneret having 8 holes. The spun polymer solution (dope) was then allowed to be coagulated by passing through a coagulation bath via a 10 mm air gap. The coagulation bath was 60 °C, and was filled with an aqueous solution having an NMP concentration of 30 % by mass. The maximum tension of the spun yarn in the coagulation bath was 0.4 g/dtex.
Then, the obtained coagulated yarn was washed by a water-washing bath adjusted to 60 °C. The fibers after washing with water were dried at 200 °C using a roller-type drying apparatus. Thereafter, hot-drawing was performed under a temperature condition of 530 °C using a non-contact type hot-drawing apparatus in which the fibers do not contact the furnace when the fibers passing through the furnace. The stretching magnification in this step was 10 times.

Finally, the stretched fibers were wound onto a paper tube by a winder at a wind ratio of 4.33 to obtain a wholly aromatic polyamide fiber having a total fineness of 25dtex, the number of filaments (the number of single yarns) of 8, and a single yarn fineness of 3.1dtex. Physical properties of the obtained wholly aromatic polyamide fiber are shown in Table 1.

< Example 2>

A wholly aromatic polyamide fiber was obtained in the same manner as in Example 1, except that a spinneret having 10 holes was used to perform an ejection so as to have a total fineness of 28 dtex, the number of filaments of 10 and a single yarn fineness of 2.8 dtex. Physical properties of the obtained wholly aromatic polyamide fiber are shown in Table 1.

< Example 3>

A wholly aromatic polyamide fiber was obtained in the same manner as in Example 1, except that a spinneret having 10 holes was used to perform an ejection so as to have a total fineness of 17 dtex, the number of filaments of 10 and a single yarn fineness of 1.7 dtex. Physical properties of the obtained wholly aromatic polyamide fiber are shown in Table 1.

< Example 4>

A wholly aromatic polyamide fiber was obtained in the same manner as in Example 1, except that a spinneret having 50 holes was used to perform an ejection so as to have a total fineness of 20 dtex, the number of filaments of 50 and a single yarn fineness of 0.4 dtex. Physical properties of the obtained wholly aromatic polyamide fiber are shown in Table 1.

< Example 5>

A wholly aromatic polyamide fiber was obtained in the same manner as in Example 1, except that the fiber was wound onto a paper tube at a wind ratio of 6.33. Physical properties of the obtained wholly aromatic polyamide fibers are shown in Table 1.

< Example 6>

A wholly aromatic polyamide fiber was obtained in the same manner as in Example 1, except that the fiber was wound onto a paper tube at a wind ratio of 2.33. Physical properties of the obtained wholly aromatic polyamide fiber are shown in Table 2.

< Example 7>

A wholly aromatic polyamide fiber was obtained in the same manner as in Example 1, except that the fiber was wound onto a paper tube at a wind ratio of 8.33. Physical properties of the obtained wholly aromatic polyamide fiber are shown in Table 2.

< Example 8>

(Preparation of polymer solution)

According to a known method, 100 parts by mass of terephthalic acid dichloride was added to 100 parts by mass of paraphenylenediamine dissolved in an NMP, a polycondensation reaction was performed, and then washing and drying were conducted to obtain a polymer of polyparaphenylene terephthalamide. The limiting viscosity (IV) of the polymer was 5.69. The obtained polyparaphenylene terephthalamide was dissolved in a concentrated sulfuric acid so as to have a concentration of 20 %.

(Production of a wholly aromatic polyamide fiber)

The polymer solution (dope) obtained above was ejected using a spinneret having 8 holes. The spun polymer solution (dope) was allowed to be coagulated by passing through a coagulation bath filled with 4 °C water via an air gap of 10 mm. The maximum tension of the spun yarn at this time was 0. 9 g/dtex. Then, the obtained coagulated yarn was appropriately neutralized with 10% by weight of an aqueous sodium hydroxide solution, and then washed with water. The fiber after washing with water was dried at 200 °C using a roller-type drying apparatus. Thereafter, the dried fiber was subjected to the hot-drawing under a temperature condition of 400 °C using a rotating roller-type hot-drawing apparatus. Finally, the stretched fiber was wound onto a paper tube by a winder at a wind ratio of 4.33 to obtain a wholly aromatic polyamide fiber having a total fineness of 25 dtex, a number of filaments of 8, and a single yarn fineness of 3.1 dtex. Physical properties of the obtained wholly aromatic polyamide fiber are shown in Table 2.

< Example 9>

A wholly aromatic polyamide fiber was obtained in the same manner as in Example 8, except that a spinneret having 10 holes was used to perform an ejection so as to have a total fineness of 28 dtex, the number of filaments of 10 and a single yarn fineness of 2.8 dtex. Physical properties of the obtained wholly aromatic polyamide fiber are shown in Table 2.

< Example 10>

A wholly aromatic polyamide fiber was obtained in the same manner as in Example 8, except that a spinneret having 10 holes was used to perform an ejection so as to have a total fineness of 17 dtex, the number of filaments of 10 and a single yarn fineness of 1.7 dtex. Physical properties of the obtained wholly aromatic polyamide fiber are shown in Table 2.

<Comparative Example 1>

A wholly aromatic polyamide fiber according to Comparative Example 1 was obtained in the same manner as in Example 1, except that a spinneret having 5 holes was used to perform an ejection so as to have a total fineness of 25 dtex, the number of filaments of 5 and a single yarn fineness of 5.0 dtex. The physical properties of the obtained wholly aromatic polyamide fiber are shown in Table 3.
As can be seen in Table 3, in the wholly aromatic polyamide fiber of Comparative Example 1, the number of fibrils increased as compared with Example 1, and deterioration in quality was observed.

<Comparative Example 2>

A wholly aromatic polyamide fiber according to Comparative Example 2 was obtained in the same manner as in Example 1, except that a spinneret having 20 holes was used to perform an ejection so as to have a total fineness of 34 dtex, the number of filaments of 20 and a single yarn fineness of 1.7 dtex. Physical properties of the obtained wholly aromatic polyamide fiber are shown in Table 3.
As can be seen in Table 3, the wholly aromatic polyamide fiber of Comparative Example 2 had an average value of the thickness of the twisted fiber of 42 $\mu$m, and had poor fiber placeability.

<Comparative Example 3>

A wholly aromatic polyamide fiber according to Comparative Example 3 was obtained in the same manner as in Example 1, except that the fiber was wound onto a paper tube at a wind ratio of 1.33. Physical properties of the obtained wholly aromatic polyamide fiber are shown in Table 3.
As can be seen in Table 3, in Comparative Example 3, the number of fibrils increased as compared with Example 1, and deterioration in quality was observed.

<Comparative Example 4>

A wholly aromatic polyamide fiber according to Comparative Example 4 was obtained in the same manner as in Example 1, except that it was wound onto a paper tube at a wind ratio of 13.33. Physical properties of the obtained wholly aromatic polyamide fiber are shown in Table 3.
As can be seen in Table 3, in Comparative Example 4, the number of fibrils increased as compared with Example 1, and deterioration in quality was observed.

<Comparative Example 5>

A wholly aromatic polyamide fiber according to Comparative Example 5 was obtained by the same manner as in Example 8, except that a contact-type hot-drawing apparatus such as a hot plate was used. The physical properties of the obtained wholly aromatic polyamide fiber are shown in Table 3.

As can be seen in Table 3, in Comparative Example 5, the number of fibrils increased as compared with Example 8, and deterioration in quality was observed.

<Comparative Example 6>

A wholly aromatic polyamide fiber according to Comparative Example 6 was obtained in the same manner as in Example 8, except that liquid flow of the coagulation bath was not controlled and the maximum tension of the spun yarn in the coagulation bath was set to 1.0 g/dtex. Physical properties of the obtained wholly aromatic polyamide fiber are shown in Table 3.

As can be seen in Table 3, in Comparative Example 6, the number of fibrils increased as compared with Example 8, and deterioration in quality was observed.

<Production of composite cable and catheter>

Whole aromatic polyamide fibers of Examples 1 to 10 and Comparative Examples 1 to 6 were used to produce composite cables and catheters. The resulting composite cables and catheters were evaluated for quality. Specifically, the thicknesses and diameters of the composite cables and catheters produced, as well as the single yarn breakage and disconnection during the production of the composite cables or catheters were evaluated. The results are shown in Table 1 and Table 2. When the quality was good, it was judged as "Good", and when the quality was poor, it was judged as "Poor".

As can be seen in Tables 1 and 2, the composite cables and the catheters, which were produced using the wholly aromatic polyamide fibers of Examples 1 to 10 having a single yarn fineness of 0.4 to 3.5 dtex, a total fineness of 5 to 30 dtex, a breaking strength of 15 cN/dtex or more, an initial tensile modulus of 500 to 750 cN/dtex, and a number of fibrils of less than 100/m, have reduced thicknesses and small diameters, and had good qualities.

On the other hand, as seen in Table 3, the composite cables and the catheters, which were produced using the wholly aromatic polyamide fibers according to Comparative Examples 1, 3, 4, 5, and 6 having the number of fibrils of 100/m or more, were poor in quality. Specifically, single yarn breakage and disconnection were occurred in the processing process.

Further, as seen in Table 3, the composite cable and the catheter produced using the wholly aromatic polyamide fiber according to Comparative Example 2 having a total fineness of 34 were also poor in quality. Specifically, the thickness was increased, and the diameter could not be reduced.

[Table 1]

Table 1

| Item | Unit | Ex.1 | Ex.2 | Ex.3 | Ex.4 | Ex.5 |
|---|---|---|---|---|---|---|
| total fineness | dtex | 25 | 28 | 17 | 20 | 25 |
| number of single yarn | filaments | 8 | 10 | 10 | 50 | 8 |
| single yarn fineness | dtex | 3.1 | 2.8 | 1.7 | 0.4 | 3.1 |
| breaking strength | cN/dtex | 25.4 | 25.5 | 25.5 | 22.7 | 25.0 |
| initial tensile modulus | cN/dtex | 626 | 655 | 652 | 672 | 617 |
| elongation at break | % | 4.0 | 3.9 | 4.6 | 3.8 | 3.9 |
| number of fibrils | fibrils/m | 26 | 18 | 22 | 27 | 43 |
| variation in single yarn diameter | % | 5.9 | 4.0 | 4.8 | 8.7 | 7.8 |

(continued)

| Item | Unit | Ex.1 | Ex.2 | Ex.3 | Ex.4 | Ex.5 |
|---|---|---|---|---|---|---|
| average value of the thickness of the twisted fiber | μ m | 30 | 31 | 24 | 23 | 31 |
| average degree of adhesion | - | 2.3 | 2.8 | 3.1 | 4.4 | 3.3 |
| maximum tension of the spun yarn in the coagulation bath | g/dtex | 0.4 | 0.4 | 0.4 | 0.4 | 0.4 |
| wind ratio | - | 4.33 | 4.33 | 4.33 | 4.33 | 6.33 |
| apparatus for hot-drawing | - | non-contact type | non-contact type | non-contact type | non-contact type | non-contact type |
| thickness | | Good | Good | Good | Good | Good |
| break at the time of the production of composite cable or catheter | | Good | Good | Good | Good | Good |

[Table 2]

Table 2

| Item | Unit | Ex.6 | Ex.7 | Ex.8 | Ex.9 | Ex.10 |
|---|---|---|---|---|---|---|
| total fineness | dtex | 25 | 25 | 25 | 28 | 17 |
| number of single yarn | filaments | 8 | 8 | 8 | 10 | 10 |
| single yarn fineness | dtex | 3.1 | 3.1 | 3.1 | 2.8 | 1.7 |
| breaking strength | cN/dtex | 24.4 | 24.4 | 22.1 | 22.4 | 23.0 |
| initial tensile modulus | cN/dtex | 616 | 622 | 694 | 708 | 711 |
| elongation at break | % | 3.8 | 3.9 | 3.2 | 3.1 | 3.0 |
| number of fibrils | fibrils/m | 66 | 51 | 42 | 39 | 52 |
| variation in single yarn diameter | % | 8.8 | 3.5 | 5.2 | 3.8 | 4.4 |
| average value of the thickness of the twisted fiber | μ m | 30 | 31 | 30 | 32 | 25 |
| average degree of adhesion | - | 2.7 | 2.5 | 1.8 | 2.2 | 2.5 |
| maximum tension of the spun yarn in the coagulation bath | g/dtex | 0.4 | 0.4 | 0.9 | 0.9 | 0.9 |
| wind ratio | - | 2.33 | 8.33 | 4.33 | 4.33 | 4.33 |
| apparatus for hot-drawing | - | non-contact type | non-contact type | roller type | roller type | roller type |
| thickness | | Good | Good | Good | Good | Good |
| break at the time of the production of composite cable or catheter | | Good | Good | Good | Good | Good |

[Table 3]

Table 3

| Item | Unit | Comp. Ex.1 | Comp. Ex.2 | Comp. Ex.3 | Comp. Ex.4 | Comp. Ex.5 | Comp. Ex.6 |
|---|---|---|---|---|---|---|---|
| total fineness | dtex | 25 | 34 | 25 | 25 | 25 | 25 |
| number of single yarn | filaments | 5 | 20 | 8 | 8 | 8 | 8 |
| single yarn fineness | dtex | 5.0 | 1.7 | 3.1 | 3.1 | 3.1 | 3.1 |
| breaking strength | cN/dtex | 23.7 | 24.7 | 17.4 | 21.7 | 16.1 | 18.2 |
| initial tensile modulus | cN/dtex | 590 | 613 | 578 | 599 | 630 | 666 |
| elongation at break | % | 3.7 | 4.0 | 3.5 | 3.7 | 3.2 | 3.2 |
| number of fibrils | fibrils/m | 121 | 83 | 500 or more | 500 or more | 200 or more | 120 |
| variation in single yarn diameter | % | 5.6 | 6.6 | 8.9 | 7.8 | 5.2 | 7.8 |
| average value of the thickness of the twisted fiber | $\mu$ m | 33 | 42 | 31 | 31 | 30 | 30 |
| average degree of adhesion | - | 3.5 | 5.6 | 3.3 | 2.3 | 1.8 | 3.2 |
| maximum tension of the spun yarn in the coagulation bath | g/dtex | 0.4 | 0.4 | 0.4 | 0.4 | 0.9 | 1.0 |
| wind ratio | - | 4.33 | 4.33 | 1.33 | 13.33 | 4.33 | 4.33 |
| apparatus for hot-drawing | - | non-contact type | non-contact type | non-contact type | non-contact type | contact type | roller type |
| thickness | | Good | Poor | Good | Good | Good | Good |
| break at the time of the production of composite cable or catheter | | Poor | - | Poor | Poor | Poor | Poor |

[REFERENCE SIGNS]

1    Metal wire
2    Wholly aromatic polyamide fiber
3    Coating resin
4    Resin layer
5    Wholly aromatic polyamide fiber
6    Paper tube
7    Cheese roll
8    Fiber

## Claims

1. A wholly aromatic polyamide fiber **characterized in that** it has a single yarn fineness of 0.4dtex to 3.5dtex, a total fineness of 5dtex to 30dtex, a breaking strength of 15cN/dtex or more, an initial tensile modulus of 500cN/dtex to 750cN/dtex, and the number of fibrils of less than 100/m,
wherein the breaking strength, the initial tensile modulus, and the number of fibrils are measured as defined in the description.

2. The wholly aromatic polyamide fiber according to claim 1, **characterized in that** it has a variation in single yarn diameter per 1m of less than 15%,
wherein the variation in single yarn diameter per 1 m is measured as defined in the description.

3. The wholly aromatic polyamide fiber according to claim 1 or 2, **characterized in that** it has an average value of the thickness of the twisted fiber of less than $40\mu m$,
wherein the average value of the thickness of the twisted fiber is an average value of the thicknesses determined by twisting the fiber the number of times calculated based on the following formula (2) using the weight calculated based on the following formula (1) and by measuring the thickness of the twisted fiber in the length direction of the fiber at five points 10cm apart:

$$\text{Weight (g)=fineness (dtex)/40} \qquad (1)$$

$$\text{Number of twists (times/m)} = 1055/\sqrt{\text{fineness (tex)}} \qquad (2).$$

4. The wholly aromatic polyamide fiber according to any one of claims 1 to 3, **characterized in that** an average degree of adhesion is 10 or less,
wherein the average degree of adhesion is measured as defined in the description.

5. The wholly aromatic polyamide fiber according to any one of claims 1 to 4, which is a polyparaphenylene terephthalamide fiber.

6. The wholly aromatic polyamide fiber according to any one of claims 1 to 4, which is a copolyparaphenylene 3, 4'-oxydiphenylene terephthalamide fiber.

7. A composite cable **characterized in that** it comprises the wholly aromatic polyamide fiber according to any one of claims 1 to 6.

8. A catheter **characterized in that** it comprises the wholly aromatic polyamide fiber according to any one of claims 1 to 6.

**Patentansprüche**

1. Vollaromatische Polyamidfaser, **dadurch gekennzeichnet, dass** sie eine Einzelgarnfeinheit von 0,4 dtex bis 3,5 dtex, eine Gesamtfeinheit von 5 dtex bis 30 dtex, eine Bruchfestigkeit von 15 cN/dtex oder mehr, einen anfänglichen Zugmodul von 500 cN/dtex bis 750 cN/dtex und die Fibrillenanzahl von weniger als 100/m aufweist,
wobei die Bruchfestigkeit, der anfängliche Zugmodul und die Fibrillenanzahl wie in der Beschreibung definiert gemessen werden.

2. Vollaromatische Polyamidfaser nach Anspruch 1, **dadurch gekennzeichnet, dass** sie eine Einzelgarndurchmesserschwankung pro 1 m von weniger als 15% aufweist,
wobei die Einzelgarndurchmesserschwankung pro 1 m wie in der Beschreibung definiert gemessen wird.

3. Vollaromatische Polyamidfaser nach Anspruch 1 oder 2, **dadurch gekennzeichnet, dass** sie einen durchschnittlichen Wert der Dicke der verdrillten Faser von weniger als 40 $\mu$m aufweist,
wobei der durchschnittliche Wert der Dicke der verdrillten Faser ein durchschnittlicher Wert der Dicken ist, die durch Verdrillen der Faser um die Anzahl von Malen, die auf Grundlage der folgenden Formel (2) unter Verwendung des Gewichts berechnet wird, das auf Grundlage der folgenden Formel (1) berechnet wird, und durch Messen der Dicke der verdrillten Faser in der Längenrichtung der Faser an fünf Punkten im Abstand von 10 cm bestimmt wird:

$$\text{Gewicht (g)} = \text{Feinheit (dtex)/40} \quad (1)$$

$$\texttt{Anzahl Verdrillungen (Male/m) = 1055/}\sqrt{\texttt{Feinheit (tex) (2)}}$$

**4.** Vollaromatische Polyamidfaser nach einem der Ansprüche 1 bis 3,

**dadurch gekennzeichnet, dass** ein durchschnittlicher Haftungsgrad 10 oder weniger beträgt,
wobei der durchschnittliche Haftungsgrad wie in der Beschreibung definiert gemessen wird.

**5.** Vollaromatische Polyamidfaser nach einem der Ansprüche 1 bis 4, die eine Polyparaphenylenterephthalamid-Faser ist.

**6.** Vollaromatische Polyamidfaser nach einem der Ansprüche 1 bis 4, die eine Copolyparaphenylen-3,4'-Oxydiphenylenterephthalamid-Faser ist.

**7.** Kompositkabel, **dadurch gekennzeichnet, dass** es die vollaromatische Polyamidfaser nach einem der Ansprüche 1 bis 6 aufweist.

**8.** Katheter, **dadurch gekennzeichnet, dass** er eine vollaromatische Polyamidfaser nach einem der Ansprüche 1 bis 6 aufweist.

## Revendications

**1.** Fibre de polyamide entièrement aromatique **caractérisée en ce qu'**elle a une finesse de fil unique de 0,4 dtex à 3,5 dtex, une finesse totale de 5 dtex à 30 dtex, une résistance à la rupture de 15 cN/dtex ou plus, un module de traction initial de 500 cN/dtex à 750cN/dtex, et un nombre de fibrilles inférieur à 100/m, la résistance à la rupture, le module de traction initial et le nombre de fibrilles étant mesurés comme défini dans la description.

**2.** Fibre de polyamide entièrement aromatique selon la revendication 1, **caractérisée en ce qu'**elle a une variation de diamètre de fil unique par 1 m inférieure à 15 %, la variation de diamètre de fil unique par 1 m étant mesurée comme défini dans la description.

**3.** Fibre de polyamide entièrement aromatique selon la revendication 1 ou 2, **caractérisée en ce qu'**elle a une valeur moyenne de l'épaisseur de la fibre torsadée inférieure à 40 μm,
dans laquelle la valeur moyenne de l'épaisseur de la fibre torsadée est une valeur moyenne des épaisseurs déterminées par torsion de la fibre le nombre de fois calculé sur la base de la formule (2) suivante en utilisant le poids calculé sur la base de la formule (1) suivante et par mesure de l'épaisseur de la fibre torsadée dans la direction de la longueur de la fibre à cinq points espacés de 10 cm :

$$\text{Poids (g)}=\text{finesse (dtex)}/40 \qquad (1)$$

$$\text{Nombre de torsions (fois/m) = 1055/}\sqrt{\text{finesse (tex)}} \qquad (2).$$

**4.** Fibre de polyamide entièrement aromatique selon l'une quelconque des revendications 1 à 3, **caractérisée en ce qu'**un degré d'adhérence moyen est de 10 ou moins,
dans lequel le degré d'adhérence moyen est mesuré tel que défini dans la description.

**5.** Fibre de polyamide entièrement aromatique selon l'une quelconque des revendications 1 à 4, qui est une fibre de polyparaphénylène téréphtalamide.

**6.** Fibre de polyamide entièrement aromatique selon l'une quelconque des revendications 1 à 4, qui est une fibre de copolyparaphénylène 3,4'-oxydiphénylène téréphtalamide.

**7.** Câble composite **caractérisé en ce qu'**il comprend la fibre de polyamide entièrement aromatique selon l'une quelconque des revendications 1 à 6.

8. Cathéter **caractérisé en ce qu'**il comprend la fibre de polyamide entièrement aromatique selon l'une quelconque des revendications 1 à 6.

[Figure 1]

## Figure 1

[Figure 2]

## Figure 2

[Figure 3]

Figure 3

## REFERENCES CITED IN THE DESCRIPTION

**Patent documents cited in the description**

- JP 2015098665 A
- EP 0051265 A1
- JP 2008156802 A
- JP H11189916 A